# EUROPÄISCHE PATENTSCHRIFT

(11) **EP 4 358 909 B1**
(45) Veröffentlichungstag und Bekanntmachung des Hinweises auf die Patenterteilung: **15.04.2026**
(21) Anmeldenummer: 22732068.6
(22) Anmeldetag: 31.05.2022
(51) Int. Cl.: A61F 13/00, A61K 9/70

(54) **VERFAHREN UND VORRICHTUNG ZUM IMPRÄGNIEREN EINER FOLIE, SOWIE VERFAHREN ZUR HERSTELLUNG EINES TRANSDERMALEN THERAPEUTISCHEN SYSTEMS**
METHOD AND APPARATUS FOR IMPREGNATION OF A FILM, AND PROCESS FOR PRODUCTION OF A TRANSDERMAL THERAPEUTIC SYSTEM
PROCÉDÉ ET APPAREIL D'IMPRÉGNATION D'UN FILM, ET PROCÉDÉ DE PRODUCTION D'UN SYSTÈME THÉRAPEUTIQUE TRANSDERMIQUE

(30) Priorität: 21.06.2021 DE 102021115977
(43) Veröffentlichungstag der Anmeldung: 01.05.2024
(73) Patentinhaber: LTS Lohmann Therapie-Systeme AG, 56626 Andernach (DE)
(72) Erfinder: STEINBORN, Peter, 56567 Neuwied (DE); SCHLÖGEL, Georg, 56626 Andernach (DE); GUTEKUNST, Dorothea, 56410 Montabaur (DE); STEINBRECHER, Viktoria, 56626 Andernach (DE); HOFFMANN, Gerd, 56566 Neuwied (DE); WIEDERSBERG, Sandra, 06268 Steigra (DE)
(74) Vertreter: dompatent
(86) Internationale Anmeldenummer: PCT/EP2022/064713
(87) Internationale Veröffentlichungsnummer: WO 2022/268450

(56) Entgegenhaltungen:
- EP-A1- 2 844 202
- EP-B1- 2 844 202
- US-A- 4 902 565

## Beschreibung

Die Erfindung betrifft ein Vorrichtungssystem zur Herstellung eines transdermalen therapeutischen Systems mit einer Vorrichtung zum Imprägnieren einer Folie und ein Verfahren zur Herstellung eines transdermalen therapeutischen Systems.

Imprägnierte Folien, insbesondere Membranen, kommen bspw. in transdermalen therapeutischen Systemen als Lage zum Einsatz.

Zum Beispiel ist in US 4 436 741 A ein transdermales therapeutisches System zur Abgabe von Scopolamin beschrieben, das eine mit Öl imprägnierte Membran aufweist.

Ein übliches Verfahren zur Imprägnierung von Folien, wie bspw. Membranen, ist das Beschichten an einer Beschichtungsanlage im Reverse-Roll-Coating mit einer Gravurwelle.

Bei der Verwendung von Gravurwalzen ist die Imprägniermenge über die Gravur der Walze, bspw. den Spalt der Gravur, bestimmt. Für jegliche Variationen der Imprägniermenge muss die Walze, bspw. die Gravurtiefe, geändert werden.

Nachteilig an bestehenden Verfahren ist u. a., dass eine ungenaue Beladung der Folie mit Imprägniermittel vorliegt.

Darüber hinaus sind bisherige Verfahren produktionstechnisch zeitaufwendig, komplex und/oder benötigen zusätzliche Arbeitsschritte und/oder Anlagen.

Weiterer druckschriftlicher Stand der Technik zu der vorliegenden Erfindung ist in EP 2 844 202 A1 und US 4 902 565 A beschrieben.

Aufgabe der Erfindung ist es, ein Verfahren zum Imprägnieren einer Folie, eine Vorrichtung zum Imprägnieren einer Folie und ein Verfahren zur Herstellung eines transdermalen therapeutischen Systems zu schaffen, wobei die Imprägnierung optimiert ist.

Die Lösung der Aufgaben erfolgt erfindungsgemäß durch ein Verfahren zur Herstellung eines transdermalen therapeutischen Systems nach Anspruch 1 und ein Vorrichtungssystem zur Herstellung eines transdermalen therapeutischen Systems nach Anspruch 2.

Bei dem erfindungsgemäßen Verfahren zur Herstellung eines transdermalen therapeutischen Systems handelt es sich insbesondere um ein Verfahren zum Herstellen eines transdermalen Pflasters oder eines Vorprodukts für ein transdermales Pflaster. Das Verfahren zur Herstellung eines transdermalen therapeutischen Systems weist die Schritte des nachfolgend beschriebenen Verfahrens zum Imprägnieren einer Folie auf. Es ist bevorzugt, dass bei der Durchführung des Verfahrens zum Imprägnieren einer Folie die nachfolgend beschriebenen Vorrichtung genutzt wird. Das Verfahren zur Herstellung eines transdermalen therapeutischen Systems weist ferner den Schritt des Hinzufügens eines Wirkstoffs in und/oder auf die imprägnierte Folie auf. Möglich ist es, dass hierbei die Folie mit dem, insbesondere flüssigen, Wirkstoff beschichtet wird.

Andererseits oder zusätzlich ist es auch möglich, dass die imprägnierte Folie mit einer oder mehrerer wirkstoffaufweisenden Folien zu einem Folienverbund verbunden wird. Der Wirkstoff weist insbesondere Scopolamin auf.

Bei dem Verfahren zum Imprägnieren einer Folie, das Teil des erfindungsgemäßen Verfahrens zur Herstellung eines transdermalen therapeutischen Systems ist, handelt es sich insbesondere um ein Verfahren zum Imprägnieren einer Membran, vorzugsweise zum Einsatz in transdermalen therapeutischen Systemen. Ein erster Schritt des Verfahrens zum Imprägnieren einer Folie besteht im Zuführen der zu der imprägnierenden Folie. Bevorzugt erfolgt das Zuführen in einem Rollverfahren, sodass die Folie von einer Zuführrolle abgewickelt wird. Die zugeführte Folie wird in ein Imprägniermittel eingetaucht. Insbesondere erfolgt ein vollständiges Eintauchen der Folie im Imprägniermittel. Das Imprägniermittel ist vorzugsweise in einem Tauchbecken, auch als Imprägnierbad zu bezeichnen, angeordnet. In bevorzugter Ausführung wird die Folie, zumindest teilweise, im Wesentlichen waagerecht durch das Imprägniermittel geführt. Bevorzugt ist es, dass die Folie nach dem Durchlaufen des Imprägniermittels vollständig mit Imprägniermittel beladen ist. Anschließend erfolgt ein teilweises Abtrennen des Imprägniermittel von der Folie. Dieses Abtrennen erfolgt insbesondere derart, dass eine vordefinierte Beladung der Folie mit Imprägniermittel erreicht wird. Insbesondere erfolgt durch das Abtrennen eine Beladung der Folie mit Öl von 5 g/m² bis 20 g/m², bevorzugt von 7 g/m² bis 13 g/m², besonders bevorzugt von ca. 10 g/m². Bevorzugt ist es, dass es sich bei dem Abtrennen um ein Abstreichen des Imprägniermittels von der Folie handelt.

Die Erfindung betrifft vorzugsweise ferner ein Vorrichtungssystem zur Herstellung eines transdermalen therapeutischen Systems. Das Vorrichtungssystem weist eine Vorrichtung zum Imprägnieren einer Folie sowie eine Wirkstoffzuführvorrichtung auf. Die Wirkstoffzuführvorrichtung ist angeordnet und/oder ausgeführt zum Hinzufügen eines Wirkstoffs in und/oder auf die imprägnierte Folie. Möglich ist es, dass hierbei die Folie mit dem, insbesondere flüssigen, Wirkstoff beschichtet wird. Andererseits oder zusätzlich ist es auch möglich, dass die imprägnierte Folie mit einer oder mehrerer wirkstoffaufweisenden Folien zu einem Folienverbund verbunden wird. Der Wirkstoff weist insbesondere Scopolamin auf.

Bei der Vorrichtung zum Imprägnieren einer Folie, die Teil des erfindungsgemäßen Vorrichtungssystems ist, handelt es sich insbesondere um eine Vorrichtung zum Imprägnieren einer Membran, vorzugsweise zum Einsatz in transdermalen therapeutischen Systemen. Die Vorrichtung weist ein mit Imprägniermittel befülltes Tauchbecken, auch als Imprägnierbad zu bezeichnen, auf. Das Tauchbecken ist zum, insbesondere durchführenden, Eintauchen der Folie in das Imprägniermittel ausgeführt und/oder angeordnet. Die Vorrichtung weist eine Folienführung auf. Die Folienführung ist insbesondere zum Zuführen der Folie, zum Durchführen der Folie, zum Eintauchen der Folie in das Tauchbecken und/oder zum Ausführen der Folie ausgeführt. Zuführen der Folie meint insbesondere ein Führen der Folie in die Vorrichtung, Durchführen der Folie meint insbesondere eine Führung der Folie innerhalb der Vorrichtung, Eintauchen der Folie meint insbesondere ein Führen der Folie durch das Tauchbecken, und Ausführen der Folie meint insbesondere ein Führen der Folie aus der Vorrichtung heraus. Es ist bevorzugt, dass zumindest teilweise innerhalb des Tauchbeckens mindestens eine, insbesondere rotierbare Umlenkrolle zum Führen der Folie angeordnet ist. Besonders bevorzugt ist es, dass mindestens zwei, insbesondere exakt zwei, Umlenkrollen innerhalb des Tauchbeckens derart angeordnet sind, dass die Folie im Wesentlichen waagrecht, zumindest durch einen Teil des Tauchbeckens führbar ist. Ferner ist es bevorzugt, dass die Folienführung mindestens eine, insbesondere rotierbare, Rolle, bspw. eine Umlenkrolle aufweist. Die mindestens eine Rolle ist vorzugsweise rund ausgeführt. Es ist bevorzugt, dass mindestens eine Rolle angetrieben ist. Bevorzugt weist die Folienführung eine Zuführrolle zum Abwickeln der Folie und/oder eine Ausführrolle zum Aufwickeln der Folie auf. Mindestens eine der Zuführrolle und der Ausführrolle sind insbesondere angetrieben ausgeführt. Die mindestens eine Rolle, insbesondere die Zuführrolle und/oder Ausführrolle, können vorzugsweise außerhalb, innerhalb oder teilweise außerhalb und innerhalb der Vorrichtung angeordnet sein. Die Vorrichtung weist darüber hinaus eine Trennvorrichtung zum, insbesondere teilweisen, Abtrennen des Imprägniermittel von der Folie auf. Die Trennvorrichtung ist vorzugsweise derart ausgeführt, dass ein Abtrennen des Imprägniermittel zur Erreichung einer vordefinierten Beladung der Folie mit Imprägniermittel erfolgt.

Beim erfindungsgemäßen Verfahren ist es bevorzugt, dass das Abtrennen des Imprägniermittels mittels einer Trennvorrichtung erfolgt. Die Trennvorrichtung die im Rahmen des Verfahrens eingesetzt wird ist insbesondere ausgeführt wie die Trennvorrichtung der erfindungsgemäßen Vorrichtung.

In bevorzugter Ausführung weist die Trennvorrichtung mindestens einen Abstreifer auf. Der Abstreifer weist vorzugsweise eine Silikonbeschichtung auf. Insbesondere ist der Abstreifer mit einer Silikonfolie von vorzugsweise 0,8 mm bis 3 mm Dicke beschichtete, bevorzugt beklebt. Es ist bevorzugt, dass der Abstreifer derart ausgeführt und/oder angeordnet ist, dass er mit der mit Imprägniermittel beladenen Folie in Kontakt kommt, vorzugsweise daran angedrückt wird. Der Abstreifer ist insbesondere druckbelastet. Es ist bevorzugt, dass die Trennvorrichtung das Imprägniermittel auf einer Seite der Folie oder auf beiden Seiten der Folie abtrennt.

In bevorzugter Ausführung weist die Trennvorrichtung mindestens eine Kante und/oder mindestens eine, vorzugsweise rotierbare, Rolle auf. Die Kante weist vorzugsweise eine eckige oder runde ist Struktur auf. Es ist bevorzugt, dass die Kante derart ausgeführt und/oder angeordnet ist, dass Kante, mit der relativ zur Kante einlaufenden oder auslaufenden Folie in Kontakt kommt. Mit anderen Worten ist es bevorzugt, dass die Kante einen spitzen oder stumpfen oder rechtwinkligen Winkel zur Folie bezogen auf die Folienlaufrichtung aufweist. Es ist bevorzugt, dass die Trennvorrichtung mindestens ein Andruckblech aufweist, das die Kante ausbildet. Das Andruckbleck ist insbesondere biegbar.

In bevorzugter Ausführung ist der Abstreifer beidseitig der mit Imprägniermittel beladenen Folie angeordnet.

In bevorzugter Ausführung kommt der Abstreifer beidseitig mit der Folie, insbesondere andrückend, in Berührung. Es ist bevorzugt, dass der mindestens eine Abstreifer in beidseitiger Ausführung zwei gegenüberliegende Rollen aufweist. Andererseits kann der beidseitige Abstreifer zwei gegenüberliegende Kanten, oder eine Kante und eine gegenüberliegende Andrückfläche, bspw. eines Ambosses aufweisen.

In bevorzugter Ausführung ist die Trennvorrichtung, insbesondere der mindestens eine Abstreifer, variabel einstellbar ausgeführt ist, sodass durch das Abtrennen von Imprägniermittel die vordefinierte Beladung der Folie mit Imprägniermittel erreichbar ist. Es ist somit bevorzugt, dass die Trennvorrichtung, insbesondere der mindestens eine Abstreifer, derart ausgeführt ist, dass eine gewünschte Beladung, bspw. eine vordefinierte Menge Öl in Litern oder Kilogramm pro Folienfläche in Quadratmetern, erreichbar ist.

In bevorzugter Ausführung weist die die Trennvorrichtung zur Einstellung der vordefinierten Beladung mindestens eine Stellvorrichtung auf. Die Stellvorrichtung weist insbesondere eine der folgenden Vorrichtungen auf, oder besteht daraus: eine Pneumatikvorrichtung, eine Hydraulikvorrichtung, eine Federvorrichtung, ein Elastomer, eine Andruckschraube, und ein Feinverstellungsgewinde. Diese Stellvorrichtung ist insbesondere angeordnet und/oder ausgeführt zur Einstellung eines Drucks, mit dem der mindestens eine Abstreifer auf die Folie drückt und/oder eines Abstands den der mindestens einen Abstreifer zur Folie aufweist. Bei einer beidseitigen Ausführung des mindestens einen Abstreifer ist die Stellvorrichtung insbesondere angeordnet und/oder ausgeführt zur Einstellung eines Drucks und/oder Abstands zwischen den beiden gegenüberliegenden Elementen, bspw. Rollen, Kanten oder Andrückfläche, des Abstreifers.

Die Federvorrichtung weist insbesondere eine Druck- oder Zugfeder, insbesondere in Ausführung einer Spiralfeder auf. Bei dem, insbesondere Gummi aufweisenden, Elastomer handelt es sich insbesondere um ein elastisches Band. Bevorzugt ist es, dass ein Anstellwinkel des Abstreifers, insbesondere in Ausführung mit Kante, verstellbar ist. Bspw. ist es vorzugsweise umgesetzt, dass der Winkel der Kante, bspw. des Andruckblechs, relativ zu einer gegenüberliegenden Kante oder zu einer gegenüberliegenden Andrückfläche veränderbar ist. Durch die Veränderbarkeit des Abstreifers sind insbesondere verschiedene Beladungszustände einstellbar. Möglich ist es bevorzugt, dass bei Abstreifer in beidseitiger Ausführung, die gegenüberliegenden Elemente versetzt oder parallel zueinander ausgeführt sind. Es ist bevorzugt, dass ein Versatz der gegenüberliegenden Elemente einstellbar ist.

Es ist bevorzugt, dass die Trennvorrichtung einen oder mehrere der oben beschriebenen Abstreifer, insbesondere in verschiedenen Ausführungen, aufweist.

In bevorzugter Ausführung ist die Trennvorrichtung, zumindest teilweise, derart ausgeführt und/oder angeordnet, dass das abgetrennte Imprägniermittel in das Tauchbecken zurückgeführt wird. Vorzugsweise ist die Trennvorrichtung, zumindest teilweise, derart ausgeführt und/oder angeordnet, dass das abgetrennte Imprägniermittel aufgrund der Schwerkraft in das Tauchbecken fällt. Hierbei ist es bevorzugt, dass die Trennvorrichtung, zumindest teilweise, über dem Tauchbecken angeordnet ist. Das erfindungsgemäße Verfahren weist, insbesondere zusätzlich oder alternativ zu dieser Ausführung der Trennvorrichtung, den Schritt der, vorzugsweise schwerkraftbedingten, Rückführung, zumindest Teilen des abgetrennten Imprägniermittels, auf. Bevorzugt ist es, dass die Folie, bspw. durch die Folienführung, nach Verlassen des Tauchbeckens im Wesentlichen senkrecht geführt wird.

In bevorzugter Ausführung weist die Vorrichtung eine, vorzugsweise mit dem Tauchbecken verbundene, Auffangwanne zum Auffangen des abgetrennten Imprägniermittels auf. Die Auffangwanne ist vorzugsweise fluidführend mit dem Tauchbecken verbunden. Andererseits ist es bevorzugt, dass die Auffangwanne separat zum Tauchbecken ausgeführt ist. Bevorzugt ist es, dass die Auffangwanne größer, insbesondere flächenmäßig, ausgestaltet ist als das Tauchbecken. Vorzugsweise decken das Tauchbecken und die Auffangwanne, insbesondere vollständig den Bereich unterhalb der Trennvorrichtung ab, sodass abgetrenntes, bspw. herunter tropfendes, Imprägniermittel aufgefangen wird.

In bevorzugter Ausführung handelt es sich bei dem Imprägniermittel um Öl, insbesondere Mineralöl. Das Imprägniermittel ist insbesondere hautverträglich.

In bevorzugter Ausführung weist die Folie Polypropylen auf, besteht insbesondere daraus. Die Folie ist insbesondere porös, vorzugsweise mikroporös, ausgestaltet. Bei der Folie handelt es sich insbesondere um Celgard^{®} 2400 des Unternehmens Celgard.

In bevorzugter Ausführung weist das erfindungsgemäße Verfahren den weiteren Schritt auf: Durchführen einer Prüfung, insbesondere Prozesskontrolle, der mit Imprägniermittel beladenen Folie. Der Schritt erfolgt insbesondere nach dem Abtrennen des Imprägniermittels zur Umsetzung der gewünschten Beladung. Bei der Prüfung erfolgt insbesondere ein Feststellen der vorliegenden Beladung. Diese vorliegende Beladung entspricht insbesondere einer IST-Beladung. Bevorzugt erfolgt zusätzlich ein Vergleich der IST-Beladung mit einer SOLL-Beladung. Es ist bevorzugt, dass zusätzlich der Schritt Einstellung der Trennvorrichtung, insbesondere zur Anpassung der IST-Beladung an die SOLL-Beladung erfolgt. Die Prüfung erfolgt insbesondere optische und/oder gewichtserfassend. Es ist bevorzugt, dass zur Prüfung ein Teil der Folie entnommen, bspw. ausgeschnitten, wird und sodann dieses Teilstück geprüft wird. Die Prüfung kann vorzugsweise mit der nachstehend beschriebenen Prüfvorrichtung durchgeführt werden.

In bevorzugter Ausführung weist die Vorrichtung eine Prüfvorrichtung zur Prüfung der mit Imprägniermittel beladenen Folie auf. Die Prüfvorrichtung ist insbesondere in Folieführrichtung hinter der Trennvorrichtung angeordnet. Die Prüfvorrichtung weist vorzugsweise eine Waage zum Wiegen der Folie auf. Zusätzlich oder alternativ weist die Prüfvorrichtung insbesondere mindestens eine optische Kontrollvorrichtung, wie bspw. eine Kamera, auf. Die Prüfvorrichtung kann bspw. auch magnetisch oder elektrisch ausgestaltet sein, sodass z. B. die Leitfähigkeit der Folie messbar ist.

Ein, insbesondere letzter, Schritt des Verfahrens zum Imprägnieren einer Folie besteht vorzugsweise in einem Ausführen der imprägnierten Folie. Das Ausführen erfolgt insbesondere in einem Rollverfahren, sodass die imprägnierte Folie oder ein die imprägnierte Folie aufweisender Folienverbund, auf eine Ausführrolle aufgewickelt wird. Die Zuführrolle und/oder die Ausführrolle kann bevorzugt angetrieben sein.

Insbesondere sind die Merkmale, die im Rahmen Verfahrens zum Imprägnieren einer Folie beschrieben sind, auch auf die Vorrichtung anwendbar, und umgekehrt. Das Verfahren zur Herstellung eines transdermalen therapeutischen Systems wird vorzugsweise mit einer oben beschriebenen Vorrichtung durchgeführt.

Bei dem Verfahren zur Herstellung eines transdermalen therapeutischen Systems handelt es sich insbesondere um ein Endlosverfahren.

Imprägniert meint im Rahmen dieser Anmeldung insbesondere eine Beladung einer, insbesondere porösen Folie, mit einem Imprägniermittel, wie Öl.

Ein weiterer Vorteil der Erfindung ist, dass der Arbeitsschritt der Imprägnierung, insbesondere beim Einsatz der Folie in transdermalen therapeutischen Systemen innerhalb einer Konfektionieranlage bzw. während des Konfektionierens erfolgen kann, anstatt wie bisher im Stand der Technik als zusätzlicher Beschichtungsarbeitsschritt bzw. innerhalb einer Beschichtungsanlage.

Nachfolgend wird die Erfindung anhand bevorzugter Ausführungsformen unter Bezugnahme auf die anliegenden Zeichnungen näher erläutert.

Es zeigen:
- Fig. 1: eine schematische Seitenansicht einer Ausführungsform der erfindungsgemäßen Vorrichtung,
- Fign. 2a-2c: schematische Darstellungen von Alternativen Abstreifern in Anlehnung an den Detailausschnitt II aus Fig. 1, und
- Fig. 3: eine perspektivische Seitenansicht einer weiteren Ausführungsform der erfindungsgemäßen Vorrichtung.

Ähnliche oder identische Bauteile oder Elemente werden in den Figuren mit den gleichen Bezugszeichen bzw. Variationen davon (z.B. 12 und 12', 12" oder 14 und 14', 14") identifiziert.

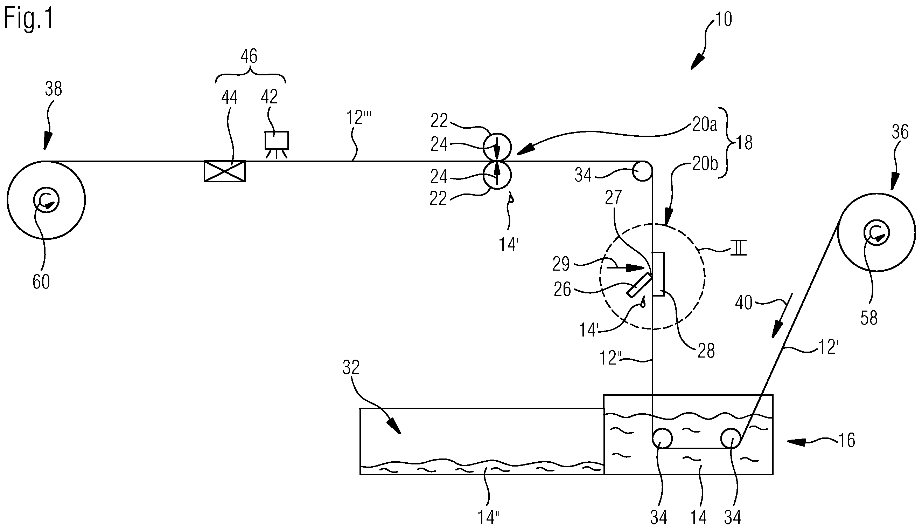

Figur 1 zeigt eine Ausführungsform einer erfindungsgemäßen Vorrichtung 10 zum Imprägnieren einer Folie 12.

Die, vorzugsweise poröse, Folie 12 wird von einer Zuführrolle 36 abgewickelt, wobei die Zuführrolle 36 gegen den Uhrzeigersinn dreht, dargestellt durch Pfeil 58. Die Folie 12 wird demnach in Richtung des Pfeils 40 geführt.

Die noch nicht mit Imprägniermittel 14 beladenen Folie 12 ist mit Bezugszeichen 12' identifiziert, während die beladene Folie 12 Bezugszeichen 12" aufweist.

Die Folie 12' wird zunächst in ein mit Imprägniermittel 14 befülltes Tauchbecken 16 geführt und dort, vorzugsweise vollständig mit Imprägniermittel 14 beladen. Innerhalb des Tauchbeckens 16 sind zwei Umlenkrollen 34 derart angeordnet, dass die Folie 12 im Wesentlichen waagrecht durch das Imprägniermittel 14 im Tauchbecken 16 läuft. Die beladenen Folie 12" tritt anschließend aus dem Tauchbecken 16 aus.

Die Folie 12" wird sodann in einen ersten Abstreifer 20b einer Trennvorrichtung 18 geführt. Hierbei wird die Folie 12" von dem Andruckblech 26 mit Kante 27 gegen die Andrückfläche 28 gedrückt (dargestellt durch Pfeil 29). Teile des Imprägniermittels 14 werden hierdurch von der Folie 12" abgetrennt. Dargestellt ist dies beispielhaft durch den Tropfen 14' aus abgetragenem Imprägniermittels 14. Aufgrund der Anordnung des Abstreifers 20b relativ zum Tauchbecken 16 fällt das abgetragenen Imprägniermittel 14 zurück in das Tauchbecken 16 und kann erneut verwendet werden.

Über die Umlenkrolle 34 wird die Folie 12" weiter in einen weiteren Abstreifer 20a der Trennvorrichtung 18 geführt. Dieser Abstreifer 20a weist zwei gegenüberliegende Rollen 22 auf. Es ist bevorzugt, dass zumindest eine der Rollen 22, insbesondere beide Rollen 22, druckbeaufschlagt (dargestellt durch Pfeile 24) sind, sodass ein Druck auf die Folie 12" ausgeübt wird. Durch den Abstreifer 20a erfolgt ein weiteres Abtragen von Imprägniermittel 14 von der Folie 12", beispielhaft dargestellt durch den Tropfen 14'. Das abgetrennte Imprägniermittel 14' fällt hierbei in eine Auffangwanne 32, die das Imprägniermittel 14" sammelt.

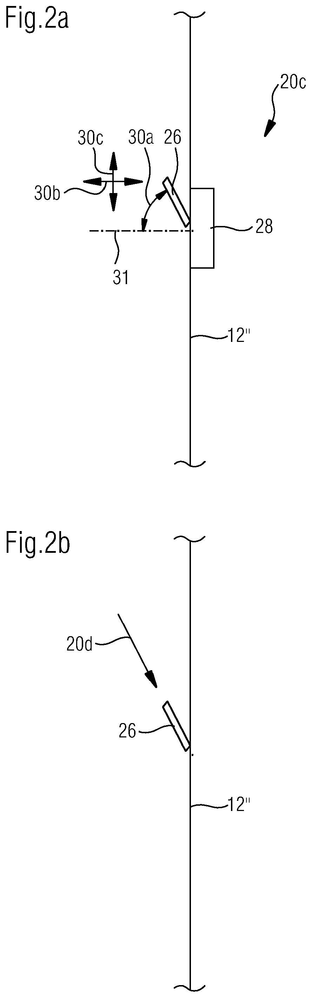

Durch die Trennvorrichtung 18 wird insbesondere eine vordefinierte Beladung der Folie 12 mit Imprägniermittel 14 umgesetzt. Die dargestellte Ausführung der Trennvorrichtung 18 mit Abstreifern 20a, 20b stellt hierbei nur eine bevorzugte Möglichkeit dar. Es sind Ausführungen der Trennvorrichtung 18 mit einem oder mehreren Abstreifern; unterschiedlichen Abstreiferausführungen (siehe bspw. Fign. 2a-2c); unterschiedlichen Anordnungen der Abstreifer; und/oder unterschiedliche Stellvorrichtungen für die Abstreifer möglich. Die Stellvorrichtungen der Abstreifer 20a, 20b ist in Figur 1 nur schematisch durch die Pfeile 24 für Abstreifer 20a sowie durch Pfeil 29 für Abstreifer 20b dargestellt. Bevorzugt ist es, dass die Stellvorrichtung einstellbar ausgeführt ist. Die Stellvorrichtung kann bspw. eine Pneumatikvorrichtung, eine Hydraulikvorrichtung, eine Federvorrichtung, ein Elastomer, eine Andruckschraube, und/oder ein Feinverstellungsgewinde aufweisen. Durch die Stellvorrichtung kann vorzugsweise der Abstand und/oder der Winkel und/oder der Druck zwischen den Abstreiferelementen, dargestellt zwischen Rollen 22 oder Andruckblech 26 und Andrückfläche 28, verändert werden.

Durch die Umlenkrollen 34 sowie die Zuführrolle 36 und die Ausführrolle 38 ist dargestellt eine Folienführung umgesetzt. Auch hierbei sind verschiedene Variationen möglich. Zuführrolle 36 und/oder Ausführrolle 38 können angetrieben sein.

Nachdem die Folie den dargestellt Abstreifer 20a passiert hat liegt insbesondere ein Folie 12‴ in einem gewünschten Beladungszustand vor.

Die Folie 12‴ wird anschließend in einer Prüfvorrichtung 46 geprüft. Insbesondere wird der Beladungszustand der Folie 12‴ geprüft, vorzugsweise gemessen. Dargestellt weist die Prüfvorrichtung 46 eine Waage 44 zum Wiegen der Folie 12‴ auf. Insbesondere wird ein Flächenabschnitt der Folie 12‴ gewogen. Ferner weist die Prüfvorrichtung 46 dargestellt eine optische Prüfvorrichtung 42, wie bspw. eine Kamera auf. Durch eine Beladung der Folie 12‴ verändert sich generell das Aussehen der Folie 12‴. So wird bspw. die Folie 12‴ mit zunehmender Beladung mit Imprägniermittel durchsichtiger. Dies kann von der optischen Prüfvorrichtung 42 erfasst werden. Anstelle der dargestellten Prüfvorrichtung 46 ist auch eine andere Ausführung möglich, bspw. eine Prüfvorrichtung 46 mit nur einer Waage 44 oder nur einer optischen Prüfvorrichtung 42. Auch ist eine ansonsten identische Ausführung der Vorrichtung 10 ohne Prüfvorrichtung 46 möglich.

Die Folie 12‴ wird anschließend auf der Ausführrolle 38 aufgewickelt. Die Ausführrolle 38 dreht sich dargestellt gegen den Uhrzeigersinn (Pfeil 60).

Möglich ist es, dass vor und/oder nach dem Aufwickeln der Folie 12‴ ein Wirkstoff, bspw. Scopolamin, mit der Folie 12‴ verbunden wird. Einerseits kann hierzu eine weitere Folienlage mit Wirkstoff mit der Folie 12‴ verbunden werden, und/oder der Wirkstoff wird direkt, bspw. als Beschichtung, mit der Folie 12‴ verbunden. Hierdurch kann ein transdermales therapeutisches System hergestellt werden.

Figuren 2a-2c zeigen beispielhaft alternative Ausführungen eines Abstreifers 20c, 20d, 20e. Diese Abstreifer 20c, 20d, 20e können bspw. anstelle des Abstreifers 20b an der Position II in Figur 1 eingesetzt werden.

Figur 2a zeigt einen Abstreifer 20c mit einer Andrückfläche 28 und einem gegenüber angeordneten Andruckblech 26. Das Andruckblech 26 ist hierbei, insbesondere relativ zur Andrückfläche 28, verstellbar ausgeführt. Dargestellt kann bspw. der Winkel (Pfeil 30a) des Andruckblechs 26 geändert werden. Möglich ist bspw. ein Winkel zwischen +90° und -90° ausgehend von der Hilfslinie 31. Bevorzugt ist ein eine Einstellbarkeit mit einem Winkel zwischen +45° und -45°. Andererseits ist dargestellt auch der Abstand (Pfeil 30b) des Andruckblechs 26 zur Andrückfläche 28 einstellbar. Hierdurch kann bspw. der Druck verändert werden. Dies kann bspw. mittels einer Federvorrichtung und/oder einer Einstellschraube erfolgen. Ferner ist dargestellt, dass ein Versatz (Pfeil 30c) zwischen Andruckblech 26 und Andrückfläche 28 einstellbar ist. In alternativen, bevorzugten Ausführungen ist nur eins oder mehrere der Einstellmöglichkeiten vorhanden. Auch ist es bevorzugt möglich die Einstellmöglichkeiten bei anderen Abstreiferausführungen umzusetzen.

Figur 2b zeigt einen einseitigen Abstreifer 20d mit einem Andruckblech 26, dass in Kontakt mit der Folie 12" gebracht wird und hierdurch Imprägniermittel abträgt.

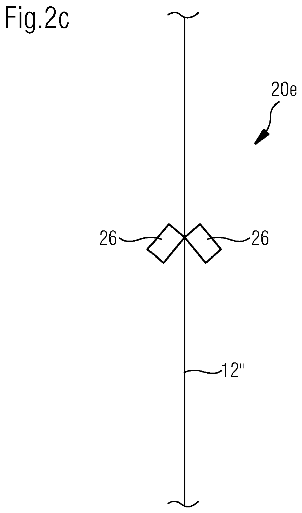

Figur 2c zeigt eine weitere Ausführung eines Abstreifer 20e, wobei zwei gegenüberliegende, schräg angeordnete Andruckbleche 26 beidseitig mit der Folie 12" und/oder dem Imprägniermittel in Berührung kommen und somit Imprägniermittel abtragen.

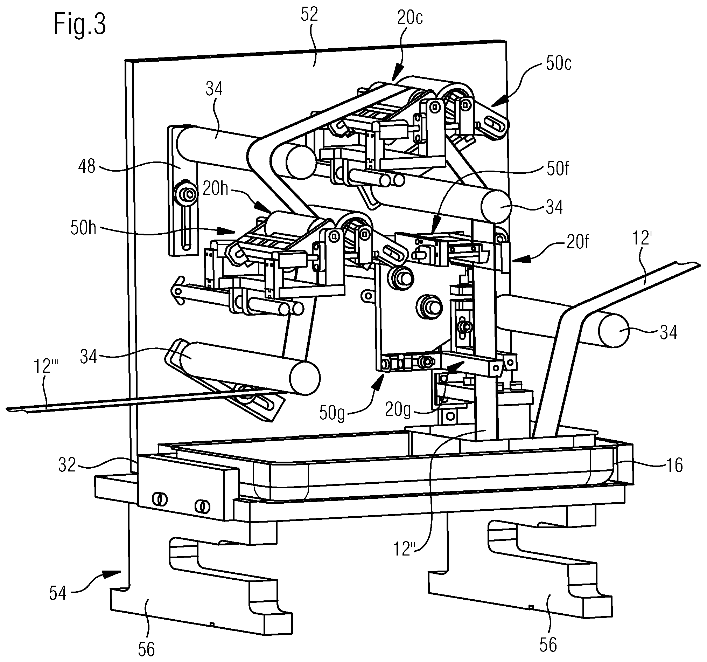

Figur 3 zeigt eine weiter Ausführungsform einer erfindungsgemäßen Vorrichtung 10 zum Imprägnieren einer Folie 12. Die Vorrichtung aus Figur 3 ist zumindest teilweise vergleichbar ausgeführt wie die Vorrichtung 10 aus Figur 1 (siehe oben).

Die Vorrichtung 10 weist eine über eine, zwei Füße 56 aufweisende, Standvorrichtung 54 angeordnete Befestigungsplatte 52 auf, an der zumindest einige der Komponenten der Vorrichtung 10 angebracht sind.

Die Folie 12' wird in der Figur von rechts kommen zugeführt und links, Folie 12‴, ausgeführt.

Die Vorrichtung 10 weist mehrere Abstreifer 20e-20h einer Trennvorrichtung auf, mit denen Imprägniermittel von der Folie 12' abgetragen wird.

Die Abstreifer 20e, 20h sind als Rollenpaar-Abstreifer ausgeführt, wobei es bevorzugt ist, dass der Druck mittels Stellvorrichtungen 50e, 50h einstellbar ist.

Der Abstreifer 20e weist ein Andruckblechs 26 und eine Andrückfläche 28 auf, wobei insbesondere der Winkel des Andruckblechs 26 variierbar ist.

Der Abstreifer 20f weist zwei Andruckbleche 26 auf, die dargestellt leicht versetzt angeordnet sind. Auch eine entsprechende Anordnung ohne Versatz oder mit einem größeren Versatz ist möglich. Ferner ist es möglich, dass der Winkel eines oder beider Andruckbleche 26 variierbar ist.

Die Umlenkrollen 34 sind insbesondere über Einstellvorrichtungen 48 variierbar.

Das erfindungsgemäße Verfahren zur Herstellung eines transdermalen therapeutischen Systems kann vorzugsweise mit einem oder mehreren Merkmalen der Figuren 1-3 durchgeführt werden.

## Patentansprüche

1. Verfahren zur Herstellung eines transdermalen therapeutischen Systems, mit dem Schritt
des Imprägnierens einer Folie (12), wobei das Imprägnieren der Folie (12) folgende Schritte aufweist:
- Zuführen der zu imprägnierenden Folie (12);
- Eintauchen der Folie (12) in ein, in einem Tauchbecken (16) angeordnetem, Imprägniermittel (14); und
- teilweises Abtrennen, insbesondere Abstreichen, des Imprägniermittels (14) von der Folie (12) zur Erreichung einer vordefinierten Beladung der Folie (12) mit Imprägniermittel (14);
wobei das Verfahren ferner den Schritt des Hinzufügens eines Wirkstoffs in und/oder auf die Folie (12) aufweist.

2. Vorrichtungssystem zur Herstellung eines transdermalen therapeutischen Systems, mit
einer Vorrichtung (10) zum Imprägnieren einer Folie (12), wobei die Vorrichtung (10) aufweist:
ein, mit Imprägniermittel (14) befüllten, Tauchbecken (16);
eine Folienführung zum Zuführen einer zu imprägnierenden Folie (12), Eintauchen der Folie (12) in das Tauchbecken (16) und Ausführen der Folie (12); und
eine Trennvorrichtung (18) zum Abtrennen des Imprägniermittels (14) von der Folie (12) zur Erreichung einer vordefinierten Beladung der imprägnierten Folie (12) mit Imprägniermittel (14),
**dadurch gekennzeichnet, dass**
das Vorrichtungssystem ferner eine Wirkstoffzuführvorrichtung zum Hinzufügen eines Wirkstoffs in und/oder auf die imprägnierte Folie (12) aufweist.

3. Verfahren nach Anspruch 1, **dadurch gekennzeichnet, dass** das Abtrennen des Imprägniermittels (14) mittels einer Trennvorrichtung (18) erfolgt.

4. Verfahren nach Anspruch 3 oder Vorrichtungssystem nach Anspruch 2, **dadurch gekennzeichnet, dass** die Trennvorrichtung (18) mindestens einen, vorzugsweise silikonbeschichteten, Abstreifer aufweist.

5. Verfahren nach oder Vorrichtungssystem Anspruch 4, **dadurch gekennzeichnet, dass** der Abstreifer mindestens eine Kante und/oder mindestens eine Rolle aufweist.

6. Verfahren oder Vorrichtungssystem nach Anspruch 4 oder 5, **dadurch gekennzeichnet, dass** der, insbesondere druckbelastete, Abstreifer beidseitig der mit Imprägniermittel (14) beladenen Folie (12) angeordnet ist.

7. Verfahren nach einem der Ansprüche 3-6 oder Vorrichtungssystem nach einem der Ansprüche 2 oder 4-6, **dadurch gekennzeichnet, dass** die Trennvorrichtung (18) variabel einstellbar ausgeführt ist,
sodass durch das Abtrennen von Imprägniermittel (14) die vordefinierte Beladung erreichbar ist.

8. Verfahren nach Anspruch 7 oder Vorrichtungssystem nach Anspruch 7, **dadurch gekennzeichnet, dass** die Trennvorrichtung (18) mindestens eine Stellvorrichtung zur Einstellung eines Drucks und/oder eines Abstands, insbesondere des Abstreifers, wobei die Stellvorrichtung vorzugsweise aufweist: eine Pneumatikvorrichtung, eine Hydraulikvorrichtung, eine Federvorrichtung, ein Elastomer, eine Andruckschraube, und ein Feinverstellungsgewinde.

9. Verfahren nach einem der Ansprüche 3-8 oder Vorrichtungssystem nach einem der Ansprüche 2 oder 4-8, **dadurch gekennzeichnet, dass** die Trennvorrichtung (18) derart angeordnet ist, dass abgetrenntes Imprägniermittel (14) in das Tauchbecken (16) zurückgeführt wird, insbesondere in das Tauchbecken (16) fällt.

10. Verfahren nach einem der Ansprüche 1 oder 3-9 oder Vorrichtungssystem, **gekennzeichnet durch** eine, vorzugsweise mit dem Tauchbecken (16) verbundenen, Auffangwanne zum Auffangen des abgetrennten Imprägniermittels (14).

11. Verfahren nach einem der Ansprüche 1 oder 3-10 oder Vorrichtungssystem nach einem der Ansprüche 2 oder 4-10, **dadurch gekennzeichnet, dass** das Imprägniermittel (14) Öl, bevorzugt Mineralöl, aufweist, insbesondere daraus besteht.

12. Verfahren nach einem der Ansprüche 1 oder 3-11 oder Vorrichtungssystem nach einem der Ansprüche 2 oder 4-11, **dadurch gekennzeichnet, dass** die Folie (12) eine, insbesondere Polypropylen aufweisende, poröse Folie (12) ist.

13. Verfahren nach einem der Ansprüche 1 oder 3-12, **dadurch gekennzeichnet, dass** das Verfahren den weiteren Schritt aufweist:
- Durchführen einer, insbesondere optischen und/oder gewichtserfassenden, Prozesskontrolle zur Prüfung der mit Imprägniermittel (14) beladenen Folie (12).

14. Verfahren nach einem der Ansprüche 1 oder 3-13 oder Vorrichtungssystem nach einem der Ansprüche 2 oder 4- 12, **gekennzeichnet durch** eine, insbesondere eine Waage (44) und/oder eine optische Kontrollvorrichtung (42) aufweisende, Prüfvorrichtung (46) zur Prüfung der der mit Imprägniermittel (14) beladenen Folie (12).

15. Verfahren nach einem der Ansprüche 1 oder 3-14 oder Vorrichtungssystem nach einem der Ansprüche 2, 4-12 oder 14, **dadurch gekennzeichnet, dass** beim Hinzufügen des Wirkstoffs die Folie (12) mit einem, insbesondere flüssigen, Wirkstoff beschichtet wird und/oder die Folie (12) mit einer oder mehreren wirkstoffaufweisenden Folien zu einem Folienverbund verbunden wird.

## Claims

1. A method for manufacturing a transdermal therapeutic system, comprising the step of:
impregnating a film (12), wherein the impregnation of the film (12) comprises the following steps:
- feeding the film (12) to be impregnated;
- immersing the film (12) into an impregnating agent (14) arranged in an immersion basin (16); and
- partially separating, in particular wiping off, the impregnating agent (14) from the film (12) in order to achieve a predefined loading of the film (12) with impregnating agent (14);
the method further comprising the step of adding an active ingredient into and/or onto the film (12).

2. A device system for manufacturing a transdermal therapeutic system, comprising
a device (10) for impregnating a film (12), the device (10) comprising:
an immersion basin (16) filled with impregnating agent (14);
a film guiding for feeding a film (12) to be impregnated, immersing the film (12) into the immersion basin (16), and feeding the film (12) out; and
a separating device (18) for separating the impregnating agent (14) from the film (12) in order to achieve a predefined loading of the impregnated film (12) with impregnating agent (14).
**characterized in that**
the device system further comprises an active ingredient supply device for adding an active ingredient into and/or onto the impregnated film (12).

3. The method according to claim 1, **characterized in that** the separation of the impregnating agent (14) is effected by means of a separating device (18).

4. The method according to claim 3 or the device system according to claim 2, **characterized in that** the separating device (18) comprises at least one wiper, preferably coated with silicone.

5. The method or the device system according to claim 4, **characterized in that** the wiper comprises at least one edge and/or at least one roller.

6. The method or the device system according to claim 4 or 5, **characterized in that** the in particular pressurized wiper is arranged on both sides of the film (12) loaded with impregnating agent (14).

7. The method according to any one of claims 3-6 or the device system according to any one of claims 2 or 4-6, **characterized in that** the separating device (18) is configured to be variably adjustable
so that the predefined loading be achieved by separating the impregnating agent (14).

8. The method according to claim 7 or the device system according to claim 7, **characterized in that** the separating device (18) comprises at least one adjusting device for adjusting a pressure and/or a distance, in particular of the wiper, the adjusting device preferably comprising: a pneumatic device, a hydraulic device, a spring device, an elastomer, a pressure screw and a fine adjustment thread.

9. The method according to any one of claims 3-8 or the device system according to any one of claims 2 or 4-8, **characterized in that** the separating device (18) is arranged such that the separated impregnating agent (14) is returned to the immersion basin (16), in particular falls into the immersion basin (16).

10. The method according to any one of claims 1 or 3-9 or the device system, **characterized by** a collecting tray, preferably connected to the immersion basin (16), for collecting the separated impregnating agent (14).

11. The method according to any one of claims 1 or 3-10 or the device system according to any one of claims 2 or 4-10, **characterized in that** the impregnating agent (14) comprises oil, preferably mineral oil, in particular consists thereof.

12. The method according to any one of claims 1 or 3-11 or the device system according to any one of claims 2 or 4-11, **characterized in that** the film (12) is a porous film (12), in particular comprising polypropylene.

13. The method according to any one of claims 1 or 3-12, **characterized in that** the method comprises the following further step:
- performing in particular an optical and/or weight-recording process control for testing the film (12) loaded with impregnating agent (14).

14. The method according to any one of claims 1 or 3-13 or the device system according to any one of claims 2 or 4-12, **characterized by** a testing device (46), in particular comprising a scale (44) and/or an optical control device (42), for testing the film (12) loaded with impregnating agent (14).

15. The method according to any one of claims 1 or 3-14 or the device system according to any one of claims 2, 4-12 or 14, **characterized in that** when the active ingredient is added, the film (12) is coated with an, in particular liquid, active ingredient and/or the film (12) is combined with one or more films containing active ingredients to form a film composite.

## Revendications

1. Procédé de production d'un système thérapeutique transdermique avec l'étape d'imprégnation d'un film (12), l'imprégnation du film (12) comprenant les étapes suivantes :
- faire amener le film (12) à imprégner ;
- plonger le film (12) dans un produit d'imprégnation (14) disposé dans une cuve de plongée (16) ;
- enlever partiellement, notamment racler, le produit d'imprégnation (14) du film (12) pour obtenir une charge prédéterminée du film (12) avec le produit d'imprégnation ;
le procédé comprenant en outre l'étape d'ajouter un principe actif dans et/ou sur le film (12).

2. Système de dispositifs pour la production d'un système thérapeutique transdermique avec
un dispositif (10) pour l'imprégnation d'un film (12) le dispositif (10) comprenant :
une cuve de plongée (16) remplie d'un produit d'imprégnation (14) ;
un guidage de film pour faire amener un film (12) à imprégner, plonger le film (12) dans la cuve de plongée (16) et faire sortir le film (12) ; et
un dispositif d'enlèvement (18) pour séparer le produit d'imprégnation (14) du film (12) pour obtenir une charge prédéterminée du film (12) avec le produit d'imprégnation (14),
**caractérisé en ce que**
le système de dispositifs comprend en outre un dispositif d'amené de principe actif pour ajouter un principe actif dans et/ou sur le film (12) imprégné.

3. Procédé selon la revendication 1, le raclage du produit d'imprégnation (14) étant effectué moyennant un dispositif d'enlèvement (18).

4. Procédé selon la revendication 3 ou système de dispositifs selon la revendication 2, **caractérisé en ce que** le dispositif d'enlèvement (18) comprend au moins une racle, de préférence recouverte de silicone.

5. Procédé ou système de dispositifs selon la revendication 4, **caractérisé en ce que** la racle comprend au moins un bord et/ou au moins un rouleau.

6. Procédé système de dispositifs selon la revendication 4 ou 5, **caractérisé en ce que** la racle, étant notamment sous pression, est disposée des deux côtés du film (12) chargé d'un produit d'imprégnation (14).

7. Procédé selon l'une des revendications 3 à 6 ou système de dispositifs selon l'une des revendications 2 ou 4 à 6, **caractérisé en ce que** le dispositif d'enlèvement (18) est conçu de façon à pouvoir être réglé de façon variable,
de sorte que la charge prédéfinie puisse être obtenue par l'enlèvement de produit d'imprégnation (14).

8. Procédé selon la revendication 7 ou système de dispositifs selon la revendication 7, **caractérisé en ce que** le dispositif d'enlèvement (18) comprend au moins un dispositif de réglage pour régler une pression et/ou une distance, notamment de la racle, le dispositif de réglage comprenant de préférence : un dispositif pneumatique, un dispositif hydraulique, un dispositif à ressort, un élastomère, une vis d'appui et un filetage de réglage fin.

9. Procédé selon l'une des revendications 3 à 8 ou système de dispositifs selon l'une des revendications 2 ou 4 à 8, **caractérisé en ce que** le dispositif d'enlèvement (18) est disposé de façon que du produit d'imprégnation (14) enlevé soit reconduit, notamment tombe, dans la cuve de plongée (16).

10. Procédé selon l'une des revendications 1 ou 3 à 9 ou système de dispositifs, **caractérisé par** une cuve de réception, de préférence reliée à la cuve de plongée (16), pour recevoir le produit d'imprégnation (14) enlevé.

11. Procédé selon l'une des revendications 1 ou 3 à 10 ou système de dispositifs selon l'une des revendications 2 ou 4 à 10, **caractérisé en ce que** le produit d'imprégnation (14) comprend de l'huile, de préférence de l'huile minérale, ou en est fait.

12. Procédé selon l'une des revendications 1 ou 3 à 11 ou système de dispositifs selon l'une des revendications 2 ou 4 à 11, **caractérisé en ce que** le film (12) est un film (12) poreux, comprenant notamment du polypropylène.

13. Procédé selon l'une des revendications 1 ou 3 à 12, **caractérisé en ce que** le procédé comprend l'étape supplémentaire :
- effectuer un contrôle de procédé, notamment optique et/ou saisissant le poids, pour examiner le film (12) chargé d'un produit d'imprégnation (14).

14. Procédé selon l'une des revendications 1 ou 3 à 13 ou système de dispositifs selon l'une des revendications 2 ou 4 à 12, **caractérisé par** un dispositif de contrôle (46), comprenant notamment une balance (44) et/ou un dispositif de contrôle optique (42), pour contrôler le film (12) chargé du produit d'imprégnation (14).

15. Procédé selon l'une des revendications 1 ou 3 à 14 ou système de dispositifs selon l'une des revendications 2, 4 à 12 ou 14, **caractérisé en ce que**, lors de l'ajout du principe actif, le film (12) est recouvert d'un principe actif, notamment liquide, et/ou le film (12) est assemblé avec un ou plusieurs films comprenant du principe actif, pour former un assemblage de film.
